# EUROPEAN PATENT APPLICATION

(11) **EP 3 733 100 A1**
(43) Date of publication of application: **04.11.2020**
(21) Application number: 18897021.4
(22) Date of filing: 28.12.2018
(51) Int. Cl.: A61B 17/70

(54) **SPINAL FIXATION SYSTEM**

(30) Priority: 28.12.2017 JP 2017254037
(71) Applicant: MIZUHO Corporation, Tokyo 113-0033 (JP)
(72) Inventor: EBARA Sohei, Chigasaki-shi, Kanagawa 253-0013 (JP)
(74) Representative: SSM Sandmair
(86) International application number: PCT/JP2018/048481
(87) International publication number: WO 2019/132005

(57) **Abstract**

To provide a spinal fusion system that applies a stable fixing force to a spine.

In a spinal fusion system 1A, a lamina having relatively high strength in an entire vertebra can be engaged to be held from a head side and a tail side by a pair of first and second hook members 3 and 4. Consequently, a fixing force to a spine can be improved and a stable fixing force can be applied to the spine.

## Description

### Technical field

The present invention relates to, for example, a spinal fusion system for correcting and fixing spinal deformity.

### Background Art

In its normal condition, a spine is generally straight when viewed from the back, cervical vertebrae and lumbar vertebrae curve forward when viewed from the side, and thoracic vertebrae and sacral vertebrae curve backward. Accordingly, the spine shows an approximately S-shaped appearance.

Spinal deformity includes diseases such as scoliosis or kyphosis. Scoliosis is the disease in which a spine is curved laterally and twisted. Kyphosis is the disease in which the angle of thoracic kyphosis becomes extremely large, or lumbar lordosis is lost so as to be deformed toward kyphosis.

In the treatment of such types of spinal deformities, spinal deformity correction and fusion surgeries are generally conducted. Spinal deformity correction and fusion surgeries are operations for first correcting deformed spines and returning them to a normal state or a state closer thereto and then fixing the deformed spines. Spinal deformity correction and fusion surgeries involve a posterior correction and fusion surgery or an anterior correction and fusion surgery. In particular, the posterior correction and fusion surgery is conducted as follows. A patient is positioned on an operation table in a prone position. Then, an operative wound or a percutaneous operative wound using minimally invasive techniques is placed in the exact middle of the patient's back, and the posterior elements of the spine are unfolded. Subsequently, a later-explained spinal deformity correction and fusion system is installed to the spine so as to three-dimensionally correct the spinal deformity. The spine is fixed in that condition.

As conventional spinal deformity correction and fusion systems, for example, Patent Literature 1 discloses a spinal fusion system. The spinal fusion system is provided with: a plurality of screws that are to be screwed into a vertebral body through the pair of pedicles of each vertebra of a spine; a crosslink that is to be connected with each screw in such a manner as to bridge each screw; and a rod that extends along an axial direction and is connected with each crosslink. Moreover, in this spinal fusion system, a set screw is adopted to connect the screw with the crosslink, and the crosslink with the rod. Specifically, when the screw is connected with the crosslink, the crosslink is engaged with the top-opened groove portion of the screw. Then, with the set screw, the crosslink is pressed against the bottom surface of the top-opened groove portion of the screw so as to connect the screw with the crosslink. As the same manner, when the crosslink is connected with the rod, the rod is engaged with the top-opened groove portion of the crosslink. Then, with the set screw, the rod is pressed against the bottom surface of the top-opened groove portion of the crosslink so as to connect the crosslink with the rod.

### Prior Art

### Patent Literature

Patent Literature 1: Japanese Patent No. 6192661

### Summary of Invention

### Problems to be Solved by Invention

However, in the conventional spinal fusion system explained above, when an external force acts on the rod, there is a concern about loosening of the rod in a sliding direction with respect to the crosslinks and loosening in a coming-off direction of the screw with respect to the vertebral body. Stable correction and fusion (a fixing force) does not act on the spine. The spinal fusion system lacks reliability in the correction and fusion.

The present invention has been devised in view of such a point, and an object of the present invention is to provide a spinal fusion system that applies a stable fixing force to a spine.

### Solution to Problem

### (Aspects of Invention)

Aspects of the invention described below illustrate the configuration of the present invention and are divided into items in order to facilitate understanding of various configurations of the present invention. The items do not limit the technical scope of the present invention, and constituent elements obtained by substituting or deleting a part of constituent elements of the items or adding other constituent items to the constituent elements while considering a best mode for carrying out the invention are also included in the technical scope of the present invention.

(1) A spinal fusion system including: a pair of hook members engageable to hold a lamina from a head side and a tail side; and a rod connectable to the hook members and extending along an axial direction of a spine (equivalent to the invention of claim 1).
   In the spinal fusion system described in the item (1), the lamina having relatively high strength in an entire vertebra is engaged to be held from the head side and the tail side by the pair of hook members to fix the spine. Therefore, a stable fixing force can be applied to the spine. In an operative procedure, when a screw generally included as a spinal fusion system is screwed into a vertebral body via a pedicle of a vertebra, high accuracy is required with respect to a screwing direction of the screw. In the spinal fusion system described in the item (1), the fixing force to the spine can be improved without including the screw. Therefore, the operative procedure can be simplified and a surgery time can be considerably reduced.
(2) In the spinal fusion system described in the item (1), the pair of hook members is connected to be capable of approaching and separating from each other (equivalent to the invention of claim 2).
   In the spinal fusion system described in the item (2), in surgery, the lamina can be easily engaged to be held from the head side and the tail side by the pair of hook members. As a result, the operative procedure using the spinal fusion system can be facilitated.
(3) In the spinal fusion system described in the item (1) or the item (2), the hook member includes: a rod receiving portion including a groove portion that receives the rod; and a hook portion connectable to the rod receiving portion and engageable with the lamina, and the hook portion is configured to be fixable to the rod receiving portion in a state in which a distal end of the hook portion is directable in any direction, and in which the distal end faces the direction (equivalent to the invention of claim 3).
   In the spinal fusion system described in the item (3), the hook portion is coupled to the rod receiving portion with the distal end of the hook portion being directable in any direction. Therefore, in surgery, the hook portion of the hook member can be accurately engaged in an intended position of the lamina by an operator.
(4) In the spinal fusion system described in the item (3), the hook portion can be configured to be fixable to the rod receiving portion in a state in which the lamina is urged in a pushing-up direction by a distal end of the hook portion.
   In the spinal fusion system descried in the item (4), since the lamina is urged in the pushing-up direction by the distal end of the hook portion. Therefore, the fixing force to the lamina by the pair of hook members can be further improved.
(5) In the spinal fusion system described in the item (4), the pair of hook members is coupled by a shaft member to be capable of approaching and separating from each other, and a distal end of the shaft member comes into contact with a ball joint provided at a proximal end portion of the hook portion, whereby the hook portion is positioned with respect to the rod receiving portion.
   In the spinal fusion system described in the item (5), it is possible to position, with simple structure, the hook portion with respect to the rod receiving portion in the state in which the lamina is urged in the pushing-up direction by the distal end of the hook portion.
(6) The spinal fusion system described in the item (1) or the item (2) further includes a rod receiving member that is connectable to the pair of hook members and receives the rod, the rod receiving member includes: a rod receiving portion including a groove portion that receives the rod; and a supporting portion that couples the rod receiving portion and the pair of hook members, and the rod receiving portion is configured to be fixable to the supporting portion in a state in which an extending direction of the groove portion is directable in any direction, and the extending direction of the groove portion faces the direction (equivalent to the invention of claim 4).

In the spinal fusion system described in the item (6), the rod receiving portion of the rod receiving member is coupled to the supporting portion with the extending direction of the groove portion being directable in any direction. Therefore, in surgery, the rod can be easily engaged with the rod receiving portion of the rod receiving member. As a result, the operative procedure using the spinal fusion system can be facilitated.

### Advantageous Effects of Invention

In the spinal fusion system according to the present invention, since the spinal fusion system includes the pair of hook members that engages to hold the lamina having relatively high strength in the entire vertebra from the head side and the tail side. Therefore, a stable fixing force can be applied to the spine.

### Brief Description of Drawings

Fig. 1 is a rear view of a state in which laminas of vertebrae are fixed by a spinal fusion system according to the first embodiment.
Fig. 2 is a side view of the state in which the laminas of the vertebrae are fixed by the spinal fusion system according to the first embodiment.
Fig. 3 is a perspective view of first and second hook members adopted in the spinal fusion system according to the first embodiment.
Fig. 4 is a sectional view of the spinal fusion system according to the first embodiment.
Fig. 5 is a sectional view of a spinal fusion system according to the second embodiment.
Fig. 6 is a sectional view of a form in which a first hook member is fixed to a shaft member by a set screw in the spinal fusion system according to the second embodiment.
Fig. 7 is a rear view of a spinal fusion system according to the third embodiment.
Fig. 8 is a sectional view of a spinal fusion system according to the third embodiment.
Fig. 9 is a rear view of a state in which the laminas of the vertebrae are fixed by the spinal fusion system according to the first embodiment.

### Embodiments for Carrying out the Invention

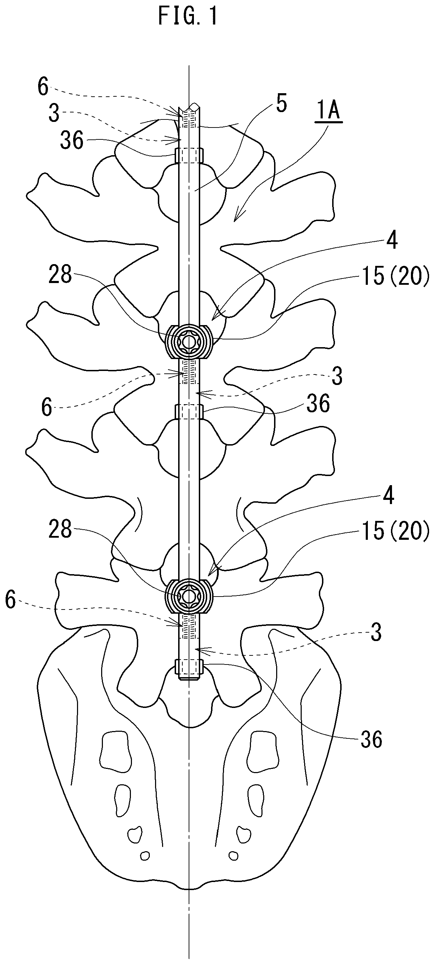

Embodiments for carrying out the present invention are explained in detail below with reference to Figs. 1 to 9.

Spinal fusion systems 1A, 1B, and 1C according to first to third embodiments of the present invention are adopted as a spinal deformity correction and fusion system for performing correction and fusion of spinal deformity such as scoliosis and kyphosis. Note that the spinal fusion systems 1A, 1B, and 1C according to the first to third embodiments can be used together with a general spinal fusion system including a plurality of screws screwed into a vertebral body via pedicles of vertebrae and a rod coupled to the plurality of screws. However, the spinal fusion systems 1A, 1B, and 1C according to the first to third embodiments can firmly fix a spine without including the screws.

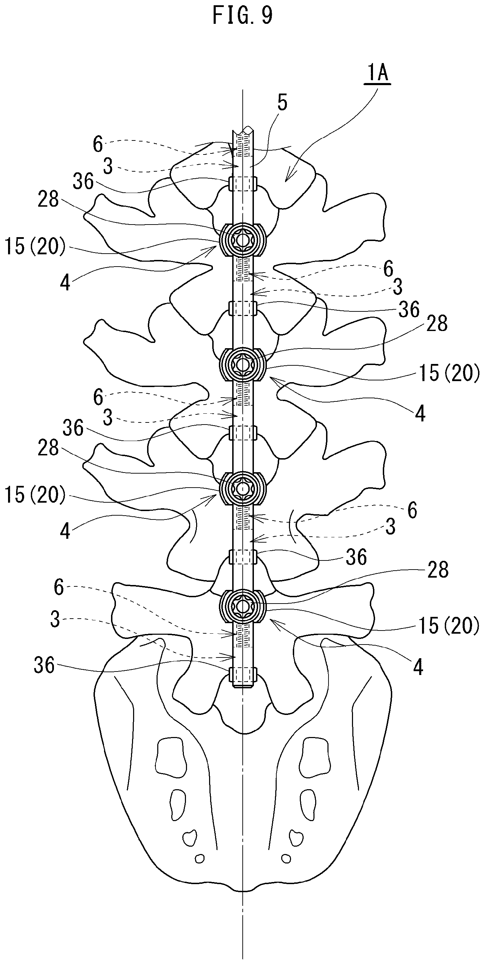

First, the spinal fusion system 1A according to the first embodiment is explained in detail with reference to Figs. 1 to 4. As illustrated in Figs. 1 to 4, the spinal fusion system 1A according to the first embodiment includes a pair of first and second hook members 3 and 4 that engages to hold laminas of the vertebrae from a head side and a tail side and a rod 5 coupled to the second hook member 4 of the pair of first and second hook members 3 and 4 and extending along an axial direction of a spine. The pair of first and second hook members 3 and 4 and the rod 5 are formed of a material rich in biocompatibility such as a titanium alloy. Note that Fig. 1 is a rear view of a state in which the laminas of the vertebrae are fixed by the spinal fusion system 1A according to the first embodiment. However, in Fig. 1, rather than the laminas of the vertebrae adjacent to one another, the laminas of the vertebrae are alternately engaged to be held by the pair of first and second hook members 3 and 4 of the spinal fusion system 1A according to the first embodiment. Naturally, as illustrated in Fig. 9, the laminas of the vertebrae adjacent to one another may be continuously engaged to be held by the pair of first and second hook members 3 and 4. In the following explanation, for convenience of explanation, a front based on a patient is referred to as one end side and a rear based on the patient is referred to as other end side.

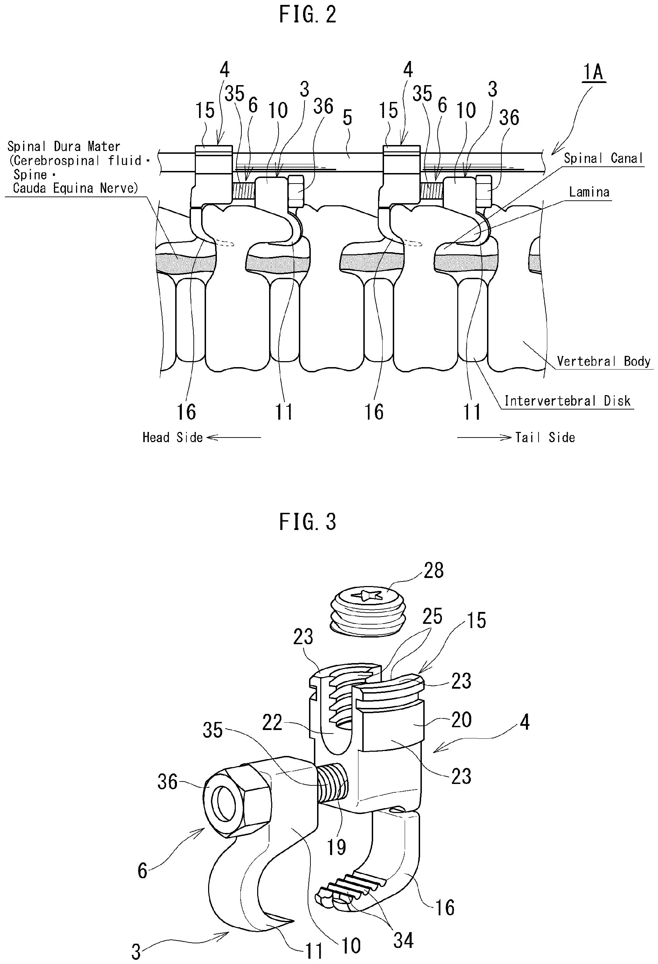

As illustrated in Figs. 2 to 4, the pair of first and second hook members 3 and 4 is coupled by a screw shaft member 6 (a shaft member) to be capable of approaching and separating from each other. The first hook member 3 includes a block-like first main body portion 10 and a first hook portion 11 extending from one end face of the first main body portion 10. A through hole 13 along the axial direction of the rod 5 is formed in the first main body portion 10. The inner diameter of the through hole 13 is set to a degree for enabling the screw shaft member 6 to move in the axial direction in the through hole 13 without the first hook member 3 causing unnecessary backlash with respect to the screw shaft member 6. The distal end of the first hook portion 11 extends in the axial direction of the through hole 13. The lamina can be engaged from the head side or the tail side (in Fig. 2, from the tail side) to be hooked from the inside of a spinal canal by the first hook portion 11. Note that various shapes may be adopted as the first hook portion 11. The shape of the first hook portion 11 is not limited if the lamina can be engaged from the head side or the tail side to be hooked from the inside of a spinal canal. The second hook member 4 includes a block-like second main body portion 15 and a second hook portion 16 extending from one end face of the second main body portion 15. The second main body portion 15 includes a female screw portion 19 formed to pierce through the screw shaft member 6 along the axial direction of the screw shaft member 6 and a rod receiving portion 20 that is provided further on the other end side than the female screw portion 19 and receives the rod 5.

A U-shaped groove portion 22 opening the other end face of the second main body portion 15 and extending in the axial direction of the rod 5 is formed in the rod receiving portion 20. The rod 5 is received in the U-shaped groove portion 22. In the rod receiving portion 20, female screw portions 25, 25 are respectively formed on the inner wall surfaces of wall portions 23, 23 opposed across the groove portion 22. A set screw 28 is screwed into the female screw portions 25, 25. The rod 5 located in the rod receiving portion 20 is pressed against the bottom surface of the groove portion 22 by the set screw 28, whereby the rod 5 is integrally coupled (fixed) to the second main body portion 15 (the rod receiving portion 20) of the second hook member 4.

In the second main body portion 15 of the second hook member 4, a spherical concaved portion 30 opening one end face of the second main body portion 15 is formed near a longitudinal direction end portion of the female screw portion 19, that is, an end portion most distant from the first hook member 3. The spherical concaved portion 30 is formed in a position where the bottom portion of the spherical concaved portion 30 overlaps the female screw portion 19. The bottom portion of the spherical concaved portion 30 is opened toward the female screw portion 19. An engagement head portion 33 (a ball joint) of the second hook portion 16 explained below is rotatably fit in the spherical concaved portion 30. The second hook portion 16 extends from the spherical concaved portion 30 toward one end side and further toward the first hook member 3 side. The lamina can be engaged from the head side or the tail side (in Fig. 2, from the head side) to be hooked from the inside of the spinal canal by the second hook portion 16. Note that various shapes may be adopted as the second hook portion 16. The shape of the second hook portion 16 is not limited if the lamina can be engaged from the head side or the tail side to be hooked from the inside of the spinal canal. As a result, the first hook portion 11 of the first hook member 3 and the second hook portion 16 of the second hook member 4 are engaged with the lamina to hold the lamina.

As explained above, the spherical engagement head portion 33 is integrally provided at the proximal end portion of the second hook portion 16. The engagement head portion 33 of the second hook portion 16 is rotatably fit in the spherical concaved portion 30 provided in the second main body portion 15. Apart of the engagement head portion 33 projects into the female screw portion 19 of the second main body portion 15 in a state in which the engagement head portion 33 of the second hook portion 16 is disposed in the spherical concaved portion 30 of the second main body portion 15. The distal end of the second hook portion 16 is coupled to the second main body portion 15 (the rod receiving portion 20) to be directable in any direction. At the distal end portion of the second hook portion 16, a plurality of uneven portions 34 extending along the width direction are formed at intervals toward the proximal end side on the inner side of the distal end portion. Consequently, a frictional force between the second hook portion 16 and the lamina is increased. Note that the second hook portion 16 of the second hook member 4 is formed slightly longer along the axial direction of the screw shaft member 6 than the first hook portion 11 of the first hook member 3. This is for the second hook portion 16 of the second hook member 4 to support to push up a wider range of the lamina compared with the first hook portion 11 of the first hook member 3.

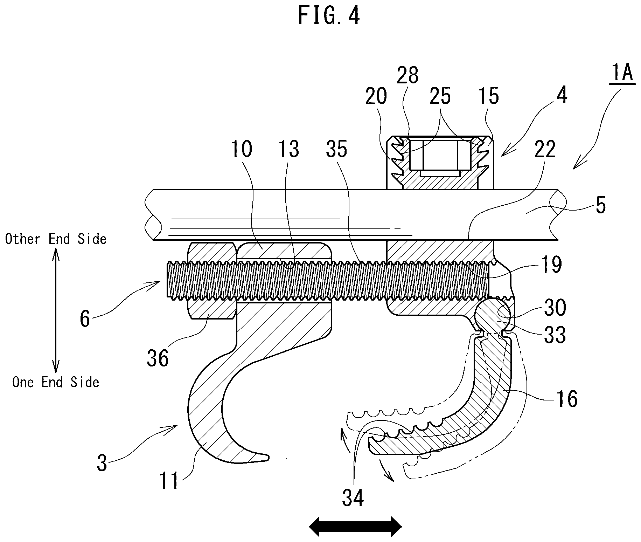

As it is seen from Fig. 4, the screw shaft member 6 includes a male screw portion 35 and an operation portion 36 integrally connected to an axial direction end portion of the male screw portion 35. The male screw portion 35 is screwed into the female screw portion 19 of the second hook member 4 (the second main body portion 15). The external shape of the operation portion 36 is formed in a polygonal shape (in this embodiment, a hexagonal shape). The male screw portion 35 of the screw shaft member 6 is inserted through the through hole 13 provided in the first main body portion 10 of the first hook member 3 and subsequently screwed into the female screw portion 19 of the second main body portion 15 of the second hook member 4. As a result, the distal end of the first hook portion 11 of the first hook member 3 and the distal end of the second hook portion 16 of the second hook member 4 are coupled in directions opposed to each other by the screw shaft member 6.

When the operation portion 36 of the screw shaft member 6 is rotated, the male screw portion 35 of the screw shaft member 6 is screwed into the female screw portion 19 of the second main body portion 15 of the second hook member 4. The screw shaft member 6 moves to the second hook member 4 side together with the first hook member 3. Therefore, the first hook member 3 and the second hook member 4 move to approach each other. When the screw shaft member 6 is further screwed, the distal end of the male screw portion 35 of the screw shaft member 6 interferes with the engagement head portion 33 provided at the proximal end portion of the second hook portion 16 projecting into the female screw portion 19 of the second hook member 4 (the second main body portion 15). As a result, while the distal end of the second hook portion 16 turns to approach the second main body portion 15, the engagement head portion 33 of the second hook portion 16 presses the inner wall surface of the spherical concaved portion 30 of the second main body portion 15, whereby the second hook portion 16 is positioned in an appropriate position and integrally coupled (fixed) to the second main body portion 15. In short, when the screw shaft member 6 is screwed into the female screw portion 19 of the second main body portion 15 of the second hook member 4 and the distal end of the screw shaft member 6 interferes with the engagement head portion 33 provided at the proximal end portion of the second hook portion 16, the second hook portion 16 is positioned with respect to the second main body portion 15 in a state in which the lamina is urged to be pushed up by the distal end of the second hook portion 16 of the second hook member 4.

Next, a method of fixing and stabilizing the spine with the spinal fusion system 1A according to the first embodiment is explained.

First, in the spine, spinous processes of the vertebrae in a fixing range of the spine are respectively cut from the roots of the spinous processes. Subsequently, from the rear of the spine, the pair of first and second hook members 3 and 4 is disposed to hold the laminas of the vertebrae from the head side and the tail side. Note that, in Figs. 1 and 2, the first hook member 3 engages to hold the lamina from the tail side and the second hook member 4 engages to hold the lamina from the head side. However, the second hook member 4 may engage to hold the lamina from the tail side and the first hook member 3 may engage to hold the lamina from the head side. At this point in time, in the second hook member 4, the second hook portion 16 is coupled to the second main body portion 15 (the rod receiving portion 20) with the distal end of the second hook portion 16 being directable in any direction. Therefore, the second hook portion 16 can be correctly engaged in an intended position of the lamina.

Subsequently, the operation portion 36 of the screw shaft member 6 is turned by a not-illustrated surgical instrument. Then, the male screw portion 35 of the screw shaft member 6 is screwed into the female screw portion 19 of the second main body portion 15 of the second hook member 4, whereby the screw shaft member 6 moves toward the second hook member 4 together with the first hook member 3. As a result, the lamina is held to be compressed from the head side and the tail side by the first hook member 3 and the second hook member 4. Thereafter, when the screwing of the screw shaft member 6 is continued, the distal end of the male screw portion 35 of the screw shaft member 6 interferes with the engagement head portion 33 of the second hook portion 16 projecting into the female screw portion 19 of the second hook member 4 (the second main body portion 15). Then, while the distal end of the second hook portion 16 turns to approach the second main body portion 15, the engagement head portion 33 of the second hook portion 16 presses the inner wall surface of the spherical concaved portion 30 of the second main body portion 15. As a result, the second hook portion 16 is positioned in a position suitable for fixing the lamina to the second main body portion 15. At this time, the distal end of the second hook portion 16 of the second hook member 4 is urged to push up a wider range of the lamina.

Subsequently, after the laminas of the vertebrae are respectively engaged to be held from the head side and the tail side by the first and second hook members 3 and 4 as explained above, the rod 5 is engaged respectively in the groove portions 22 of the rod receiving portions 20 of the second hook members 4 (the second main body portions 15).

Subsequently, the set screws 28 are screwed into the female screw portions 25, 25 of the rod receiving portions 20 of the second hook members 4. The rod 5 located in the rod receiving portions 20 of the second hook members 4 is pressed against the bottom surfaces of the groove portions 22 by the set screws 28, whereby the rod 5 is integrally coupled (fixed) to the second main body portions 15 (the rod receiving portions 20) of the second hook members 4. Note that, after the rod 5 is provisionally stopped in the groove portions 22 of the rod receiving portions 20 of the second hook members 4, appropriate operation for correcting spinal deformity such as scoliosis, kyphosis, and torsion is applied to the rod 5 and the first and second hook members 3 and 4 fixed to hold the laminas of the vertebrae.

In the spinal fusion system 1A according to the first embodiment explained above, the lamina having relatively high strength in the entire vertebra can be engaged to be held from the head side and the tail side to fix the spine by the pair of first and second hook members 3 and 4. Accordingly, a fixing force to the spine can be improved and a stable fixing force can be applied to the spine. The spinal fusion system 1A according to the first embodiment does not include a screw screwed into a vertebral body via pedicles of the vertebrae. The screw screwed into the vertebral body is required to have high accuracy in a screwing direction and the like of the screw in an operative procedure. However, screwing operation of such a screw can be eliminated, the operative procedure is further simplified, and a surgery time of the operative procedure can be greatly reduced.

In the spinal fusion system 1A according to the first embodiment, in the second hook member 4, the second hook portion is coupled to the second main body portion 15 (the rod receiving portion 20) with the distal end of the second hook portion 16 being directable in any direction. Therefore, the second hook portion 16 can be accurately engaged in an intended position of the lamina.

Further, in the spinal fusion system 1A according to the first embodiment, when the distal end of the male screw portion 35 of the screw shaft member 6 interferes with the engagement head portion 33 of the second hook portion 16 projecting into the female screw portion 19 of the second hook member 4 (the second main body portion 15), the distal end of the second hook portion 16 turns to approach the second main body portion 15, As a result, in a state in which the lamina is urged to be pushed up by the distal end of the second hook portion 16, the second hook portion 16 in the second hook member 4 is positioned and integrally coupled (fixed) to the second main body portion 15. Consequently, the fixing force for the spine by the pair of first and second hook members 3 and 4 can be further improved.

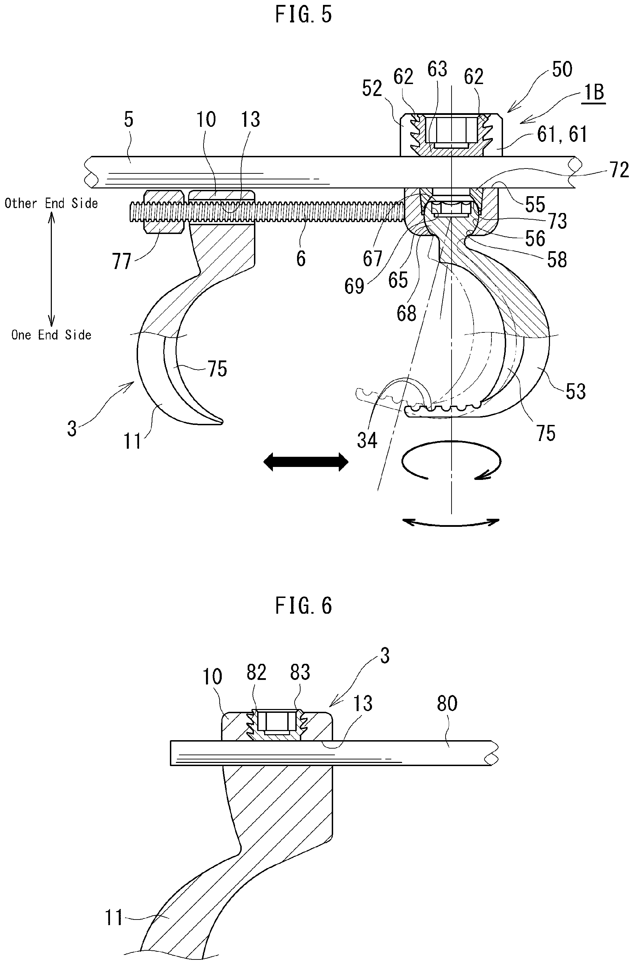

Next, the spinal fusion system 1B according to the second embodiment is explained with reference to Figs. 5 and 6. When the spinal fusion system 1B according to the second embodiment is explained, only differences from the spinal fusion system 1A according to the first embodiment are explained.

In the spinal fusion system 1B according to the second embodiment, a second hook member 50 includes a rod receiving portion 52 including a groove portion 55 that receives the rod 5 and a second hook portion 53 extending from one end face of the rod receiving portion 52. The rod receiving portion 52 is formed in a block shape. In the rod receiving portion 52, the groove portion 55 having a U shape opened on the other end face on the opposite side of the second hook portion 53 side is formed along the axial direction of the rod 5. The rod 5 is received in the groove portion 55. An insertion hole 56 is formed in the bottom portion of the groove portion 55 to pierce through the rod receiving portion 52. An engagement head portion 65 and a pressing member 72 of the second hook portion 53 explained below are engaged in the insertion hole 56. Alocking spherical surface 58 that locks a spherical surface 68 provided in the engagement head portion 65 of the second hook portion 53 is formed on the inner wall surface of the insertion hole 56.

In the rod receiving portion 52, female screw portions 62, 62 are respectively formed on the inner wall surfaces of wall portions 61, 61 opposed across the groove portion 55. A set screw 63 is screwed into the female screw portions 62, 62. The rod 5 located in the rod receiving portion 52 is moved toward the bottom portion of the groove portion 55 by the set screw 63. As a result, the pressing member 72 explained below disposed in the rod receiving portion 52 is pressed and pushed down by the set screw 63, whereby the rod 5 can be integrally coupled (fixed) to the second hook member 50 (the groove portion 55).

The second hook portion 53 extends from one end face of the rod receiving portion 52. The engagement head portion 65 is integrally connected to the proximal end portion of the second hook portion 53. The engagement head portion 65 is rotatably fit in the insertion hole 56 of the rod receiving portion 52. The engagement head portion 65 of the second hook portion 53 includes a plane portion 67 formed at the top of the engagement head portion 65 and the spherical surface 68 formed continuously from the plane portion 67. A polygonal concaved portion 69 is provided in the plane portion 67. The second hook portion 53 is configured to be fixable (integrally couplable) to the rod receiving portion 52 in a state in which the distal end of the second hook portion 53 is directable in any direction and the distal end faces any direction. A coupling structure of the second hook portion 53 is explained in detail. The pressing member 72 is engaged in the insertion hole 56 of the rod receiving portion 52 to allow sliding in the axial direction. The pressing member 72 is formed in a substantially cylindrical shape. A pressing spherical surface 73 is formed on the inner wall surface of the pressing member 72. The pressing spherical surface 73 of the pressing member 72 is brought into contact with the spherical surface 68 of the engagement head portion 65 of the second hook portion 53.

Consequently, the second hook portion 53 is coupled to the rod receiving portion 52 with the distal end of the second hook portion 53 being directable in any direction. As a result, in surgery, a dedicated surgical instrument is fit in the polygonal concaved portion 69 provided in the engagement head portion 65 of the second hook portion 53 and rotated, whereby the distal end of the second hook portion 53 can be disposed in any position. The set screw 63 is screwed into the female screw portions 62, 62 of the rod receiving portion 52 and the rod 5 is moved toward the second hook portion 53 side together with the pressing member 72. As a result, the pressing spherical surface 73 of the pressing member 72 presses the spherical surface 68 of the engagement head portion 65 of the second hook portion 53. At the same time, the spherical surface 68 of the engagement head portion 65 presses the locking spherical surface 58 of the insertion hole 56, whereby the second hook portion 53 is fixed in a state in which a direction in which the distal end is directed is positioned in any direction with respect to the rod receiving portion 52. Simultaneously with this action, the rod 5 is integrally coupled (fixed) to the rod receiving portion 52 (the groove portion 55) of the second hook member 50.

Note that a protrusion portion 75 is provided on the inner side of the second hook portion 53 of the second hook member 50 along the longitudinal direction in substantially the center in the width direction of the second hook portion 53. The protrusion portion 75 may be formed in a thin plate shape or may be formed in a triangular shape in section sharpened at the tips. The protrusion portion 75 is formed in a shape easily biting into the lamina. On the other hand, as in the second hook portion 53 of the second hook member 50, the protrusion portion 75 is projected on the inner side of the first hook portion 11 of the first hook member 3 along the longitudinal direction in substantially the center in the width direction of the first hook portion 11.

The screw shaft member 6 extends from the outer wall surface of the rod receiving portion 52 of the second hook member 50 along the axial direction of the rod 5, that is, a direction in which the groove portion 55 extends. The screw shaft member 6 is located further on one end side (the second hook portion 53 side) than the groove portion 55. The screw shaft member 6 is inserted through the through hole 13 provided in the first main body portion 10 of the first hook member 3. At this time, the distal end of the first hook portion 11 of the first hook member 3 and the distal end of the second hook portion 53 of the second hook member 50 are opposed. A nut member 77 is screwed into the longitudinal direction end portion of the screw shaft member 6. The nut member 77 is turned by a not-illustrated surgical instrument and the first hook member 3 is moved toward the second hook member 50 along the screw shaft member 6 together with the nut member 77. As a result, the lamina can be held to be compressed from the head side and the tail side by the first hook member 3 and the second hook member 50.

Note that an embodiment illustrated in Fig. 6 may be adopted. Specifically, a shaft member 80 is projected from the outer wall surface of the rod receiving portion 52 of the second hook member 50. The shaft member 80 is inserted through the through hole 13 of the first hook member 3 (the first main body portion 10)., On the other hand, a female screw portion 82 is formed on the other end lateral wall portion of the first hook member 3 (the first main body portion 10) to pierce through the other end side wall portion toward the through hole 13. A set screw 83 is screwed into the female screw portion 82. After the first hook member 3 and the second hook member 50 are disposed to hold the lamina, a compression load is applied to the first hook member 3 and the second hook member 50 in a direction in which the first hook member 3 and the second hook member 50 approach each other by a not-illustrated surgical instrument. Subsequently, while maintaining the state, the set screw 83 is screwed and the shaft member 80 is pressed against the bottom surface of the through hole 13 of the first hook member 3 (the first main body portion 10) to fix the first hook member 3 to the shaft member 80. As a result, the lamina can be held to be compressed from the head side and the tail side by the first hook member 3 and the second hook member 50.

In the spinal fusion system 1B according to the second embodiment, as in the spinal fusion system 1A according to the first embodiment, the lamina having relatively high strength in the entire vertebra can be engaged to be held from the head side and the tail side to fix the spine by the pair of first and second hook members 3 and 50. As a result, a fixing force to the spine can be improved and a stable fixing force can be applied to the spine.

In the spinal fusion system 1B according to the second embodiment, as in the spinal fusion system 1A according to the first embodiment, when the pair of first and second hook members 3 and 50 is disposed to hold the lamina from the head side and the tail side, in the second hook member 50, since the second hook portion 53 is coupled to the rod receiving portion 52 with the distal end of the second hook portion 53 being directable in any direction, the second hook portion 53 can be accurately engaged in an intended position of the lamina.

Further, in the spinal fusion system 1B according to the second embodiment, the protrusion portions 75 and 75 along the longitudinal direction are respectively projected on the inner sides of the first and second hook portions 11 and 53 of the first and second hook members 3 and 50 and in substantially the centers in the width direction of the first and second hook portions 11 and 53. When the pair of first and second hook members 3 and 50 is disposed to hold and compress the lamina from the head side and the tail side, since the protrusion portions 75 and 75 bite into the lamina, the fixing force to the lamina by the first and second hook members 3 and 50 can be further improved.

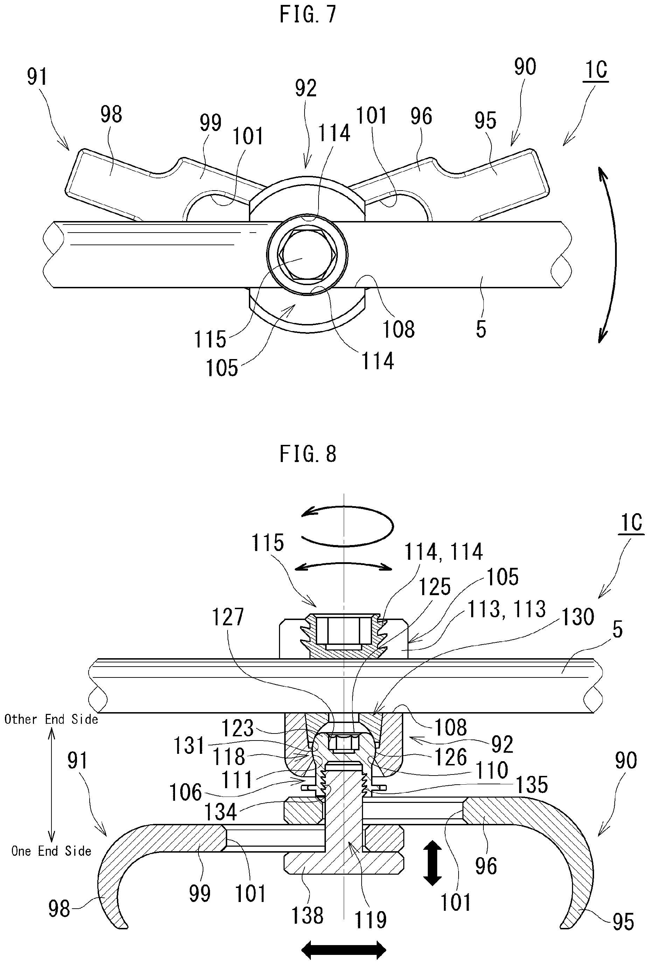

Next, the spinal fusion system 1C according to a third embodiment is explained with reference to Figs. 7 and 8. When the spinal fusion system 1C according to the third embodiment is explained, only differences from the spinal fusion system 1A according to the first embodiment are explained.

The spinal fusion system 1C according to the third embodiment includes a pair of first and second hook members 90 and 91, the distal ends of which are disposed to be opposed to each other, and a rod receiving member 92 that is coupled to the first and second hook members 90 and 91 and receives the rod 5. The first and second hook members 90 and 91 are formed in substantially the same configuration. Therefore, only the configuration of the first hook member 90 is explained and explanation of the configuration of the second hook member 91 is omitted. The first hook member 90 has a substantially rectangular shape in section and includes a first arm portion 96 extending along the axial direction of the rod 5 and a first hook portion 95 extend from the longitudinal direction end portion of the first arm portion 96 to one end side. Along hole 101 is formed in the first arm portion 96. The width of the long hole 101 of the first arm portion 96 is smaller than the outer diameter of a telescopic shaft portion 119 of a telescopic supporting portion 106 and is set to a degree enabling a plane portion provided in the telescopic shaft portion 119 to be inserted through the long hole 101 with a clearance. The lamina can be engaged from the head side or the tail side to be hooked from the spinal canal by the first hook portion 95. Note that, as illustrated in Fig. 7, in the first hook member 90, the width of the first hook portion 95 may be set smaller than the width of the first arm portion 96.

The rod receiving member 92 includes a rod receiving portion 105 including a groove portion 108 that receives the rod 5 and the telescopic supporting portion 106 including the telescopic shaft portion 119 inserted through the long holes 101, 101 of the first and second arm portions 96 and 99 of the first and second hook members 90 and 91. In the rod receiving portion 105, the groove portion 108 having a U shape opened on the other end face on the opposite side of the first and second hook members 90 and 91 side is formed along the axial direction of the rod 5. The rod 5 is received in the groove portion 108. In the bottom portion of the groove portion 108, an insertion hole 110 is formed to pierce through the rod receiving portion 105. An engagement head portion 123 and a pressing member 130 of the telescopic supporting portion 106 (a supporting main body portion 118) explained below are engaged in the insertion hole 110. On the inner wall surface of the insertion hole 110, a locking spherical surface 111 that locks a spherical surface 126 provided in the engagement head portion 123 of the telescopic supporting portion 106 (the supporting main body portion 118) is formed.

In the rod receiving portion 105, female screw portions 114, 114 are respectively formed on the inner wall surfaces of wall portions 113, 113 opposed across the groove portion 108. A set screw 115 is screwed into the female screw portions 114, 114. The rod 5 located in the rod receiving portion 105 is moved toward the bottom portion of the groove portion 108 by the set screw 115. As a result, the pressing member 130 explained below disposed in the rod receiving portion 105 is pressed and pushed down by the set screw 115, whereby the rod 5 can be integrally coupled (fixed) to the rod receiving member 92 (the groove portion 108 of the rod receiving portion 105). The telescopic supporting portion 106 extends from one end face of the rod receiving portion 105. The telescopic supporting portion 106 is formed in a stepped columnar shape as a whole. The telescopic supporting portion 106 is configured to be stretchable in the entire length thereof. Specifically, the telescopic supporting portion 106 is configured from the supporting main body portion 118 including the engagement head portion 123 and the telescopic shaft portion 119 screwed with the supporting main body portion 118 and extending to one end side and inserted through the long holes 101, 101 of the first and second arm portions 96 and 99 of the first and second hook members 90 and 91. The supporting main body portion 118 is formed in a columnar shape. The engagement head portion 123 having a spherical shape is integrally connected to the other end of the supporting main body portion 118. The engagement head portion 123 is rotatably fit in the insertion hole 110 of the rod receiving portion 105.

The engagement head portion 123 of the supporting main body portion 118 includes a plane portion 125 formed at the top of the engagement head portion 123 and the spherical surface 126 formed continuously from the plane portion 125. A polygonal concaved portion 127 is provided in the plane portion 125. The supporting main body portion 118 can be rotated by fitting a dedicated surgical instrument in the polygonal concaved portion 127 and rotating the surgical instrument. The rod receiving portion 105 is configured to be fixable (integrally couplable) to the telescopic supporting portion 106 in a state in which an extending direction of the groove portion 108 is directable in any direction and the extending direction of the groove portion 108 faces any direction. A coupling structure of the second hook portion 53 is explained in detail. The pressing member 130 is engaged in the insertion hole 110 of the rod receiving portion 105 to allow sliding in the axial direction. A pressing spherical surface 131 is formed on the inner wall surface of the pressing member 130. The pressing spherical surface 131 of the pressing member 130 is brought into contact with the spherical surface 126 of the engagement head portion 123 of the supporting main body portion 118.

Consequently, the rod receiving portion 105 is coupled to the telescopic supporting portion 106 with the extending direction of the groove portion 108 being directable in any direction. The set screw 115 is screwed into the female screw portions 114, 114 of the rod receiving portion 105. The rod 5 is moved toward the telescopic supporting portion 106 side together with the pressing member 130. As a result, the pressing spherical surface 131 of the pressing member 130 presses the spherical surface 126 of the engagement head portion 123 of the telescopic supporting portion 106 (the supporting main body portion 118). At the same time, the spherical surface 126 of the engagement head portion 123 presses the locking spherical surface 111 of the insertion hole 110, whereby the rod receiving portion 105 is coupled and fixed to the telescopic supporting portion 106 in a state in which the extending direction of the groove portion 108 is positioned in any direction. Simultaneously with this action, the rod 5 is integrally coupled (fixed) to the rod receiving portion 105 (the groove portion 108) of the rod receiving member 92.

In the supporting main body portion 118 of the telescopic supporting portion 106, a female screw portion 134 is formed from one end face of the supporting main body portion 118 at a predetermined depth. The outer diameter of the telescopic shaft portion 119 is formed smaller than the outer diameter of the supporting main body portion 118. A male screw portion 135 screwed in the female screw portion 134 of the supporting main body portion 118 is formed on the other end side of the telescopic shaft portion 119. A plane portion (not illustrated) is formed on the outer circumferential surface of the telescopic shaft portion 119 on one end side from the male screw portion 135. A disk-like locking portion 138 having an outer diameter larger than the width of the first and second arm portions 96 and 99 of the first and second hook members 90 and 91 is integrally connected to one end portion of the telescopic shaft portion 119. The telescopic shaft portion 119 of the telescopic supporting portion 106 is inserted through the long holes 101, 101 of the first and second arm portions 96 and 99 of the first and second hook members 90 and 91. The first and second arm portions 96 and 99 of the first and second hook members 90 and 91 are held to overlap between one end of the supporting main body portion 118 of the telescopic supporting portion 106 and the locking portion 138 of the telescopic shaft portion 119. As it is seen from Fig. 7, when the telescopic shaft portion 119 of the telescopic supporting portion 106 is inserted respectively through the long holes 101, 101 of the first and second arm portions 96 and 99 of the first and second hook members 90 and 91, the first and second hook members 90 and 91 becomes capable of turning within a range of a predetermined angle with respect to the telescopic supporting portion 106 by a clearance between the plane portion of the telescopic shaft portion 119 and one of opposed wall surfaces of the long hole 101. On the other hand, the telescopic shaft portion 119 of the telescopic supporting portion 106 becomes unable to rotate.

When a holding position of the lamina by the pair of first and second hook members 90 and 91 is determined, a dedicated surgical instrument is fit in the polygonal concaved portion 127 of the supporting main body portion 118 of the telescopic supporting portion 106. The surgical instrument is rotated to rotate the supporting main body portion 118. Then, the female screw portion 134 of the supporting main body portion 118 and the male screw portion 135 of the telescopic shaft portion 119 are fit and the telescopic shaft portion 119 is inserted into the long holes 101, 101 of the first and second arm portions 96 and 99 to be unable to rotate. Therefore, the telescopic shaft portion 119 moves to enter the supporting main body portion 118 (the entire length of the telescopic supporting portion 106 decreases). Consequently, the first and second arm portions 96 and 99 of the first and second hook members 90 and 91 are firmly held between one end of the supporting main body portion 118 of the telescopic supporting portion 106 and the locking portion 138 of the telescopic shaft portion 119. The positions of the first and second arm portions 96 and 99 are respectively fixed.

In the spinal fusion system 1C according to the third embodiment explained above, as in the spinal fusion system 1A according to the first embodiment, the lamina having relatively high strength in the entire vertebra can be engaged to be held from the head side and the tail side to fix the spine by the pair of first and second hook members 90 and 91. As a result, a fixing force to the spine can be improved and a stable fixing force can be applied to the spine.

In the spinal fusion system 1C according to the third embodiment, the first and second hook members 90 and 91 are rotatably supported within the range of the predetermined angle with respect to the telescopic supporting portion 106. Therefore, when the pair of first and second hook members 90 and 91 is disposed to hold the lamina from the head side and the tail side, the first and second hook portions 95 and 98 can be accurately engaged in an intended position of the lamina within the range of the predetermined angle.

Further, in the spinal fusion system 1B according to the third embodiment, in the rod receiving member 92, the rod receiving portion 105 is configured to be fixable (integrally couplable) to the telescopic supporting portion 106 in a state in which the extending direction of the groove portion 108 is directable (capable of oscillating) in any direction and the extending direction of the groove portion 108 faces any direction. Consequently, the rod 5 can be easily received in the rod receiving portions 105 (the groove portions 108) of the rod receiving members 92. The rod 5 and the rod receiving members 92 can be easily coupled and fixed.

The spinal fusion systems 1A, 1B, and 1C according to the first to third embodiments explained above are adopted as a spinal deformity correction and fusion system for fixing a relatively long range of the spine in order to perform correction and fusion of spinal deformity such as scoliosis and kyphosis. However, the spinal fusion systems 1A, 1B, and 1C according to the first to third embodiments can also be adopted as a system for fixing and stabilizing the spine in a relatively short range in other spine diseases, for example, spondylolisthesis and vertebral fracture.

### Explanation of Symbols

- 1A, 1B, 1C:: spinal fusion system
- 3, 90:: first hook member
- 4, 50, 91:: second hook member
- 5:: rod
- 6:: screw shaft member
- 11, 95:: first hook portion
- 16, 53, 98:: second hook portion
- 20, 52, 105:: rod receiving portion
- 22, 55, 108:: groove portion
- 92:: rod receiving member
- 106:: telescopic supporting portion (supporting portion)

## Claims

1. A spinal fusion system comprising:
a pair of hook members engageable to hold a lamina from a head side and a tail side; and
a rod connectable to the hook members and extending along an axial direction of a spine.

2. The spinal fusion system according to claim 1, wherein the pair of hook members is connected to be capable of approaching and separating from each other.

3. The spinal fusion system according to claim 1 or 2, wherein
the hook member includes a rod receiving portion including a groove portion that receives the rod, and a hook portion connectable to the rod receiving portion and engageable with the lamina, and
the hook portion is configured to be fixable to the rod receiving portion in a state in which a distal end of the hook portion is directable in any direction, and in which the distal end faces the direction.

4. The spinal fusion system according to claim 1 or 2, further comprising
a rod receiving member that is connectable to the pair of hook members and receives the rod, wherein
the rod receiving member includes a rod receiving portion including a groove portion that receives the rod, and a supporting portion that couples the rod receiving portion and the pair of hook members, and
the rod receiving portion is configured to be fixable to the supporting portion in a state in which an extending direction of the groove portion is directable in any direction, and in which the extending direction of the groove portion faces the direction.
